# EUROPÄISCHE PATENTSCHRIFT

(11) **EP 0 270 863 B1**
(45) Veröffentlichungstag und Bekanntmachung des Hinweises auf die Patenterteilung: **02.03.1994**
(21) Anmeldenummer: 87116624.5
(22) Anmeldetag: 11.11.1987
(51) Int. Cl.: A61F 2/36

(54) **Implantierbare Hüftgelenksprothese**
Implantable hip prosthesis
Prothèse de la hanche à implanter

(30) Priorität: 17.11.1986 DE 3639259; 04.11.1987 DE 3737372
(43) Veröffentlichungstag der Anmeldung: 15.06.1988
(73) Patentinhaber: Thull, Roger, Prof. Dr.-Ing., D-97082 Würzburg (DE)
(72) Erfinder: Thull, Roger, Prof. Dr.-Ing., D-97082 Würzburg (DE)
(74) Vertreter: Hufnagel, Walter, Dipl.-Ing., Dipl.-Wirtsch.-Ing.

(56) Entgegenhaltungen:
- DE-A- 2 726 297
- DE-A- 3 536 894
- FR-A- 2 438 468
- GB-A- 1 525 667
- Dubbel, 14. Auflage, Seite 393, Absatz 2.2.3, Tabelle 3 a

## Beschreibung

Die vorliegende Erfindung bezieht sich auf eine Hüftgelenksprothese zur zementfreien Implantation in den Markraum eines Oberschenkelknochens gemäß den Oberbegriffen der Ansprüche 1 und 3.

Eine derartige Hüftgelenksprothese ist aus der DE-A-3536894 bekannt. Mit dieser Anordnung soll eine anpassungsfähige, isoelastische Hüftgelenks-Endoprothese geschaffen werden. Der Schaft dieser bekannten Hüftgelenksprothese besteht aus wenigstens zwei an dem den Gelenkteil tragenden Flansch befestigten Stäben, die links- oder rechtsgängig derart spiralförmig verdreht sind, daß jeder Stab vom Flansch aus bis zum jeweils unteren Ende wenigstens einmal um die Zentralachse des Schaftes, also im wesentlichen in Richtung derselben, verwunden ist. Der Schaft bildet also eine Art mehrgängige Schraubenfeder mit einer sehr steilen Steigung der Windungen und mit einem relativ großen Abstand der einander benachbarten Windungen. Der Außendurchmesser des Schaftes, d.h. die durch die verdrehten Stäbe gebildete äußere Hüllkurve, ist größer bemessen als der Durchmesser des Hohlraums des Femurs an der jeweiligen Einsatzstelle. Dadurch werden beim Einsetzen des Implantats die Stäbe radial nach innen gedrückt. Hierdurch wird eine Vorspannung des Schaftes in radialer Richtung gegen die Innenwand des Femurs erzeugt. Einerseits durch diese Belastung und andererseits bei der späteren Belastung beim Gehen werden die sprialförmig verdrehten Stäbe praktisch ausschließlich auf Biegung beansprucht. Infolge der steilen Steigung der spiralförmig verdrehten Stäbe können sich diese kaum an eine regelmäßig nicht geradlinig verlaufende Höhlung des Oberschenkelknochens anpassen.

Zwar wächst das Implantat in die sich bildende neue Knochenmasse ein. Die im Vergleich zu vollen Schäften von Hüftgelenksprothesen dünnen metallischen Stäbe halten aber den extrem hohen Biegespannungen, die auch schon bei kleinen Verbiegungen im Werkstoff entstehen, wegen der zu geringen Dauerfestigkeit, die teilweise über Jahrzehnte gefordert wird, nicht stand. Darüber hinaus wird bei der bei Belastung auftretenden Biegebeanspruchung der dabei im wesentlichen am Außenbogen verlaufende Stab auf Dehnung beansprucht und der im wesentlichen am Innenbogen verlaufende Stab hingegen gestaucht. Dementsprechend wird der durch die spiralförmige, mit sehr großer Steigung ausgeführte Verwindung erhaltene Durchmesser des gedehnten Stabes verkleinert und derjenige des gestauchten Stabes vergrößert. Hierdurch werden auf die Knochenmasse fortwährend einander entgegengesetzt gerichtete, also scherende Kräfte, ausgeübt. Dies wirkt sich ebenfalls nachteilig auf die Lebensdauer der implantierten Stäbe aus.

Mit der vorliegenden Erfindung soll demgegenüber die Aufgabe gelöst werden, eine Hüftgelenksprothese der eingangs erwähnten Art derart auszubilden, daß die geschilderten Belastungen auch bei sehr langer Dauerbelastung nicht zu einer Zerstörung oder nachteiligen Ermüdung des Implantatmaterials führen. Zugleich soll eine günstige Abstimmung von Schaft und Oberschenkelknochen derart gewährleistet sein, daß Scherkräfte und Relativbewegungen an der Grenzfläche zwischen Schaft und Knochen minimiert werden. Trotzdem soll eine gute individuelle Anpassung des Schaftes an die anatomischen Verhältnisse des Markraumes möglich und es sollen durch den Schaft in den Knochen physiologisch günstige, die Vitalität des Knochens fördernde Spannungen einleitbar sein.

Gelöst wird diese Aufgabe durch die in den Kennzeichen der Ansprüche 1 und 3 angegebenen Merkmale.

Durch den geringen bzw. nicht vorhandenen Windungsabstand und die gleichzeitig geringe Steigung der Windungen bzw. der Schraubengänge des Stabes bzw. der Stäbe verläuft bzw. verlaufen die Stabachsen im Gegensatz zur bekannten Ausführung quer zur Schaftachse. Hierdurch werden der Stab bzw. die Stäbe nicht oder kaum mehr auf Biegung, sondern im wesentlichen auf Torsion beansprucht. Auch werden der Stab bzw. die Stäbe nicht mehr gedehnt oder gestaucht und auch bei der Anpassung des Schaftes an eine Biegung des Markhohlraums tritt im wesentlichen eine Torsionsbeanspruchung des Stabes bzw. der Stäbe auf. Da der Stab bzw. die Stäbe bei Torsionsbeanspruchung eine wesentlich längere Dauerbelastung aushalten, kommt es hierdurch nicht mehr zur Überbeanspruchung des Implantats. Außerdem werden die bei Steilgewinden auftretenden Wechselbelastungen der Knochenmasse vollkommen vermieden.

Weitere vorteilhafte Einzelheiten der Erfindung sind in den Unteransprüchen angegeben und werden nachfolgend anhand der in der Zeichnung veranschaulichten Ausführungsbeispiele näher beschrieben. Es zeigen:
- Fig. 1: die Hüftgelenksprothese gemäß der Erfindung in der Frontalebene im Schnitt,
- Fig. 2: die Hüftgelenksprothese nach Fig. 1 von der Seite aus gesehen,
- Fig. 3: eine Ansicht gemäß dem Schnitt A-B der Fig. 1,
- Fig. 4: eine Variante mit einer endseitigen Verankerung,
- Fig. 5: den Oberteil einer weiteren Hüftgelenksprothesenvariante mit nicht aufeinanderliegenden Windungen, die ohne Zwischenstück bis zum Kragen ausgeführt sind,
- Fig. 6: eine Ausführung mit drei gewundenen Stäben zur Bildung des Hüftgelenksprothesenschaftes,
- Fig. 7: eine weitere erfindungsgemäße Hüftgelenksprothese in der Frontalansicht,
- Fig. 8: die Seitenansicht der Hüftgelenksprothese gemäß Fig. 7 und
- Fig. 9: eine Ansicht gemäß dem Schnitt C-D der Fig. 7.

Mit 1 ist eine Hüftgelenksprothese bezeichnet, die aus einem Kragen 2, einem Hals 3, einer auf einen an den Hals 3 anschließenden Konus 4 aufsteckbaren, nicht dargestellten Hüftgelenkskugel und einem auf der Gegenseite angebrachten Schaft 5 besteht. Der Schaft 5 ragt in den Markraum 6 eines Oberschenkelknoches 7. Am Kragen 2 ist eine Auflagefläche 8 vorgesehen, die außen auf dem Oberschenkelknochen 7 aufliegt. Zur Drehfixierung des Kragens 2 kann an diesem auf der höher liegenden, von der Schaftseite 9 weiter entfernten Seite 10 ein Fixier- oder Rotationssicherungselement 11, beispielsweise in Form eines Stiftes, Flügels odgl. außerhalb des Schaftquerschnitts angebracht sein. Zusätzlich kann eine zum Konus 4 zentrale Bohrung 12 vorgesehen sein, durch die eine Verschraubung des Kragens 2 mit dem Oberschenkelknochen 7 möglich ist. Bei genügend großem Querschnitt des Fixlierelementes 11 kann auch in diesem eine Bohrung vorgenommen sein, durch die eine Verschraubung mit dem Knochen möglich ist. Auch kann das Fixierelement 11 gegebenenfalls zusätzlich oberflächenstrukturiert oder mit körperfreundlichen Werkstoffen beschichtet sein.

Erfindungsgemäß besteht der Schaft 5 aus wenigstens einem Stab 14 aus elastischem, knochenverträglichem Material, der als Spirale ausgebildet oder aus einem oder mehreren spiralförmig gewundenen Drähten besteht. Auf diese Weise erhält man einen Schaft 5 bevorzugt in Form und mit den Eigenschaften einer Spiralfeder.

Als Materialien für Hüftgelenksprothesen der hier beanspruchten Art haben sich körperverträgliche Metalllegierungen, vorzugsweise Titanlegierungen, als geeignet erweisen, insbesondere solche, die mit Aluminium, Vanadium, Eisen oder Niob legiert sind.

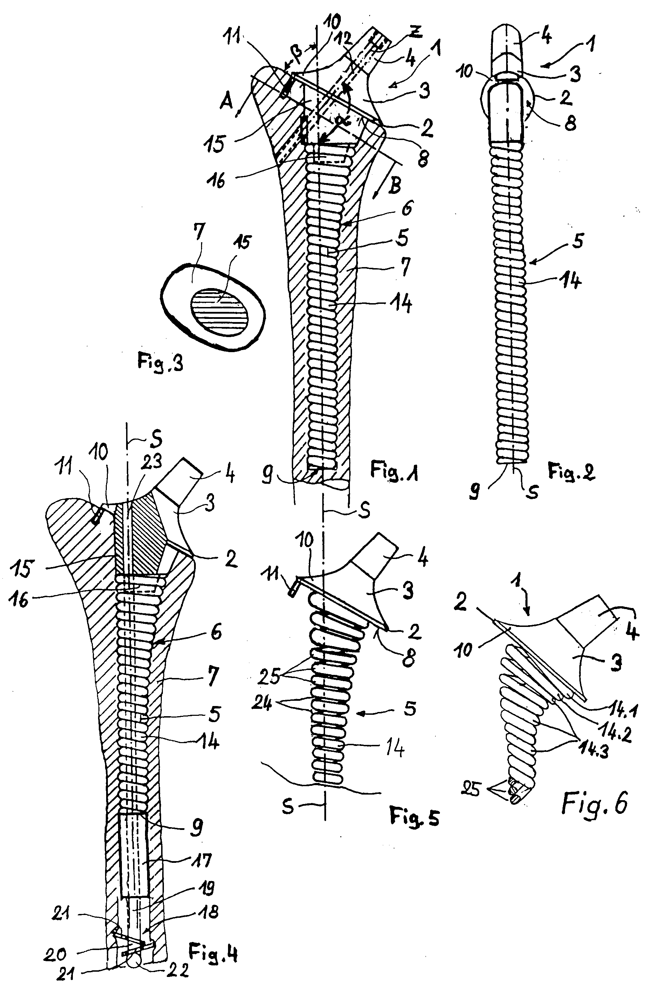

Die in Fig. 1 dargestellte Hüftgelenksprothese in Form einer Spirale zeigt aufeinanderliegende Windungen, wobei diese ohne oder mit Vorspannung gewickelt sein können. Die äußeren Konturen passen sich dem Markraum 6 des Oberschenkelknochens 7 an, wobei der Schaftumfang - wie üblich - gelenkferner (distal) geringer ist als am Gelenkansatz. Den individuell unterschiedlichen anatomischen Verhältnissen wird der Schaft durch seine Querelastizität senkrecht zur Schaftachse S gerecht. Den Gesetzen mechanischer Federn entsprechend, wird die Schaftelastizität durch den Stabdurchmesser, die elastischen Konstanten des Werkstoffs, das Wickelverhältnis und die Anzahl der federnden Windungen bestimmt und hier so eingestellt, daß die Elastizität des Schaftes quer zur Achsrichtung der des üblicherweise im Markraum befindlichen spongiösen Knochens entspricht. Eine ortsabhängige Elastizität, wie sie mitunter wünschenswert ist, wird erreicht, indem entweder das Wickelverhältnis oder der Stabdurchmesser oder beides entsprechend der Anforderungen ortsabhängig unterschiedlich gewählt wird. Ein oben steiferer Schaft 5 als unten ergibt sich beispielsweise dadurch, daß der spiralförmig gewickelte Stab 14 oben einen größeren Durchmesser aufweist als unten. Die Elastizität auf Zug kann durch den noch freien Parameter der Vorspannung beim Wickeln günstig eingestellt werden. Ohne Vorspannung werden bei Zugbelastung die Windungen voneinande weggezogen. Beim Wickeln mit Vorspannung liegen die Windungen bereits unter Spannung aufeinander, so daß eine bestimmte Zugspannung erforderlich ist, um die Windungen voneinander abzuheben.

Der obere Teil des elastischen Schaftes 5 ist angeschliffen und wird mit einem am Kragen 2 vorgesehene Zwischenstück 15 fest, vorzugsweise durch Schweißen verbunden. Dabei ist die letzte Windung des Schaftes 5 vorteilhaft so auszubilden, daß der nach dem Schweißen mechanisch stärkste Teil nach außen zu liegen kommt. Um die Stabilität gegen Knickung an der Verbindungsstelle zu erhöhen, kann zweckmäßig ein mit dem Zwischenstück 15 verbundenes Formteil 16 in den elastischen Teil des Schaftes 5 eingeführt werden. Das lateral konvex geformte Teil 16 stützt die vorzugsweise ersten beiden Windungen mechanisch ab.

Die am elastischen Schaft 5 angreifenden Kräfte und Momente richten sich nach der Größe und der Richtung der auf den Mittelpunkt der Hüftgelenkskugel wirkenden sogenannten resultierenden Kraft, also nach dem Winkel α der durch den Hals gehenden Achse Z mit der Schaftachse S

Bei einem bevorzugt angewandten Winkel α von etwa 135° bis 145° ergibt sich ein günstigster Kragenwinkel β von etwa 55° bis 65°. Zur Verankerung im kortikalen Knochen ist der lateral über den Schaftquerschnitt hinausreichende Kragen 2 mit dem als Ankerplatte ausgebildeten Fixierelement 11 versehen.

Fig. 3 zeigt, daß sich der Querschnitt des elastischen Schaftes 5 im massiven Teil desselben entsprechend fortsetzt.

Bei einer Variante der Hüftgelenksprothese gemäß der vorliegenden Erfindung findet eine zusätzliche laterale Verankerung im kortikalen Knochen durch eine Schraube oder einen hinter dem Knochen zu konternden Bolzen statt. Die Bohrung 12 zur Aufnahme einer Fixierungsschraube befindet sich in Richtung der Halsachse Z im Kopfhalsteil (Fig. 1).

Fig. 4 stellte eine weitere Variante dar, die dadurch entsteht, daß der elastische Schaft 5 am distalen Ende angeschliffen und stumpf mit einer Gewindehülse 17 verschweißt ist. In die Gewindehülse 17 wird ein Knochenanker 18 eingeschraubt. Der Knochenanker 18 besteht aus einem Gewindestift 19, auf dessen unterem gewindefreien, zylindrischen Teil 20 zwei durchbohrte Platten 21 so locker aufgeschoben sind, daß sie beim Einführen der Hüftgelenksprothese 1 in den Knochen 7 durch Aufrichten einen kleineren Durchmesser als der Markraum 6 haben. Der Knochenanker 18 wird unten durch ein kugelförmiges Endstück 22 abgeschlossen, dessen Abmessungen größer sind als die Öffnung in den Platten 21.

Die Verankerung der Prothese nach der Implantation erfolgt durch Einschrauben des Gewindestiftes 19 in die Gewindehülse 17. Hierbei verkürzt sich der Abstant zwischen dem unteren Ende der Gewindehülse 17 und den im kompakten Knochen festsitzenden Ankerplatten 21, so daß der gewickelte Stab 14 zugfederartig aufgezogen und der zwischen dem Prothesenkragen 2 und der distalen Verankerung befindliche Knochen unter eine physiologisch wünschenswerte Druckspannung gestzt wird. Die entstehenden Spannungen hängen von der sogenannten Federkonstante des Schaftes 5, dem durch Einschrauben des Gewindestiftes 19 nach der Verankerung zurückgelegten Weg und eventuellen Vorspannungen im elastischen Schaft 5 ab.

Der Gewindestift 19 wird mit Hilfe eines schlanken Werkzeugs eingeschraubt, das durch eine Bohrung 23 im Kopfhalsteil 2, 3, 16 in Verlängerung der Schaftachse S eingeführt wird.

Als besondere Vorteile der Erfindung gegenüber dem Stand der Technik lassen sich der scherkraftfreie Verbund zum Knochen hervorheben. Hieraus resultiert eine langzeitsichere Prothesenfixierung, in dem im Zustand der Ruhe unter physiologischer Druckbelastung knöchernes Gewebe in die Schaftstruktur einwächst. Die im distalen Bereich der Prothese lokalisierten Verankerungselemente sind, anders als der obere Schaftteil, nur einem vernachlässigbar kleinem Moment und damit einer Biegung ausgesetzt.

Damit kann die im elastischen Schaft gespeicherte Energie den Knochen unter Spannung halten, wodurch sich die für den Knochenanbau schädlicher Wechsel von Zug- und Druckspannungen verhindern lassen.

Eine weitere Variante der Erfindung zeigt Fig. 5, die sich von den anderen Abbildungen dadurch unterscheidet, daß der Stab 14 mit einem Abstand 24 zwischen den einzelnen Windungen 25 gewickelt ist.

Gegebenenfalls kann der massive Schaftteil 15, 16 bis zum Kragen 2 entfallen. Er wird dann durch einen in diesem oberen Endbereich im Durchmesser variablen Stab 14 ersetzt.

Der Durchmesser ist lateral größer als medial. Die angeschliffene letzte Windung kommt zweckmäßig parallel zum Kragen 2 zu liegen und sie ist mit diesem fest, etwa wiederum durch eine Schweißnaht verbunden.

Bei nach oben sich insbesondere kontinuierlich vergrößerndem Stabdurchmesser ergibt sich besonders im Biegebereich eine stärkere Federkraft. Vorzugsweise im Bereich der Biegung, also im oberen Bereich des Schaftes 5, können die Windungen außen dicker sein, indem man einen oder mehrere Stäbe 14 verwendet, die abwechselnd dünne und dicke Abschnitte aufweisen, die zweckmäßig kontinuierlich ineinander übergehen und deren Länge so bemessen ist, daß je ein dickerer und ein anschließend dünnerer Abschnitt jeweils eine Windung bilden.

In Fig. 6 ist ein Ausführungsbeispiel für eine Hüftgelenksprothese mit drei Stäben 14.1, 14.2 und 14.3 dargestellt, die durch Verwindung - entsprechend einem mehrgängigen Gewinde- eine mehrgängige Spiralfeder bilden. Auch hier können die Windungen 25 mit oder ohne Vorspannung aneinanderliegen oder sie können im Abstand voneinander angeordnet sein. Die oberen Enden sind wieder am Kragen 2 befestigt, insbesondere angeschweißt oder angegossen. Durch Verwendung mehrerer Stäbe 14.1, 14.2, 14.3 kann die Federcharakteristik, Elastizität und seitliche Bewegbarkeit weiter verändert werden. Damit ist eine weitere Möglichkeit der Anpassung an die spezifischen Gegebenheiten vorhanden.

Wesentlich für die vorliegende Erfindung ist, daß Scherkräfte zwischen dem Schaft der Hüftgelenksprothese und dem kompakten Knochen sowie Relativbewegungen zwischen den künstlichen und den natürlichen Komponenten des implantierten Hüftgelenks auf ein Minimum reduziert werden.

Vorteilhaft wird der elastisch federnde Schaft aus einem Federstab gewickelt, dessen Durchmesser in Abstimmung mit dem Wickelverhältnis der Feder, dem Schubmodul des Stabwerkstoffes, der Anzahl der federnden Windungen und einer eventuellen Vorspannung so dimensioniert ist, daß die Auslenkung senkrecht zur Schaftachse bei Belastung durch das um eine von vorn nach hinten durch die Zeichenebene gehende Achse in Richtung der Kopfhalsneigung wirkende Moment etwa derjenigen Auslenkung entspricht, die entstehen würde, wenn bei einem gesunden Hüftgelenk anstelle des elastischen Schaftes der künstlichen Hüftgelenksprothese im Markraum das üblicherweise vorhandene elastische Innenteil des kompakten Knochens vorhanden wäre.

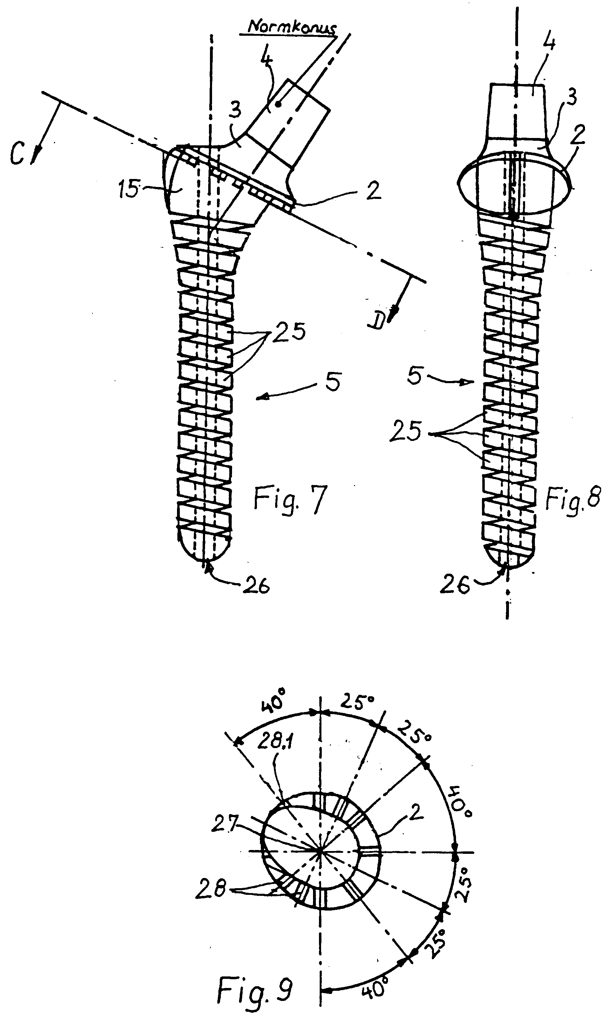

Gemäß einer anderen in den Fig. 7 und 8 dargestellten vorteilhaften Ausgestaltung der Erfindung ist der Schaft 5 als Spirale ausgebildet, die durch mechanische und/oder erosive Bearbeitung eines starrne vollen oder rohrförmigen Schaftes erzeugt ist. Bei mechanischer Bearbeitung kann der Schaft 5 zerspanend hergestellt sein, beispeilsweise durch Drehen, Fräsen oder Schleifen. Mit der Positionsziffer 26 ist die Bohrung eines rohrförmigen Schaftes bezeichnet.

Bei erosiver Herstellung ist vorzugsweise die Bearbeitung mittels Elektoerosion anwendbar. Aber auch eine Bearbeitung mit einem Flüssigkeitsstrahl, dem Schleifkörper beigefügt sind, ist möglich.

Bei diesen Herstellungsverfahren können die vorbeschriebenen unterschiedlichen Querschnitte der Sprial am Hals und zum Schaftende hin, verschiedene Steigungen der Spirale und/oder verschiedener Abstand der Windungen 25 in verschiedenen Abschnitten und/oder ein mehrgängiger Spiralenschaft aus zwei oder mehr ineinandersteckenden Spiralen verwirklicht werden. Diese Verfahren sind unter Umständen auch deshalb vorteilhafter, weil ohne große Umstände die Ausbildung der Spirale unmittelbar an einen Sonderfall angepaßt werden kann, was bei gewickelten Spiralen wegen der Notwendigkeit von Wickelformen zur Herstellung der Spiralen nicht ohne weiteres möglich ist.

Die Fig. 9 zeigt eine Möglichkeit der Anordnung mehrerer Fixierelemente 11 an der Unterseite des Kragens 2. Diese sind in Form vorzugsweise radial zu einem Zentralpunkt 27 verlaufender Stege 28, die Rotationssicherungselemente darstellen, ausgebildet. Beim Ausführungsbeispiel sind zehn solcher Stege 28 vorgesehen. Der Winkelabstand benachbarter Stege 28 kann dabei zwischen 90° und 20° gewählt werden. Im Ausführungsbiespiel sind folgende Winkelabstände, beginnend beim Steg 28.1 und im Uhrzeigersinn betrachtet:
40 - 25 - 25 - 40 - 50 - 40 - 25 - 25 - 40.

Die Höhe der Stege 28 beträgt zweckmäßig zwischen 2 mm und 6 mm.

Die Bezeichnung "spiralförmig gewundener Stab" in der vorliegenden Anmeldung bezieht sich auf einen spiralförmig verwindbaren Draht mit einem Drahtdurchmesser vorzugsweise im Bereich zwischen 3 mm und 5 mm, wobei der Drahtdurchmesser der vorstehend bezeichneten "dünneren" Windungen bevorzugt 3 mm bis 4 mm und derjenige der vorstehend bezeichneten "dickeren" Windungen vorzugsweise 4 mm bis 5 mm beträgt.

## Patentansprüche

1. Hüftgelenksprothese (1) zur zementfreien Implantation in den Markraum eines Oberschenkelknochens (7), bestehend aus einem an die Markraumkonturen anpaßbaren, elastischen, spiralförmigen Schaft (5), einem am oberen Ende des Oberschenkelknochens (7) abstützbaren Kragen (2) und einem anschließenden, einen Kugelkopf tragenden Halsstück (3), wobei der spiralförmige Schaft (5) aus mehreren in Abstand voneinander befindlichen Stäben besteht, dadurch gekennzeichnet, daß der Schaft als mehrgängige Schraubenfeder aus mehreren, jeweils eine Vielzahl von Windungen bildenden Stäben (14) oder als eingängige Schraubenfeder aus einem einzigen, eine Vielzahl von Windungen bildenden Stab (14) ausgebildet ist, wobei über die gesamte Schaftlänge der lichte Abstand zwischen den einzelnen Windungen geringer ist als die Dicke des Stabes (14) bzw. der Stäbe (14), wobei die Windungen gewickelt oder aus einem vollen Schaft herausgearbeitet sind.

2. Hüftgelenksprothese nach Anspruch 1, dadurch gekennzeichnet, daß die Windungen durch einen zerspanenden Arbeitsvorgang oder durch Elektroerosion aus dem Schaftmaterial herausgearbeitet sind.

3. Hüftgelenksprothese (1) zur zementfreien Implantation in den Markraum eines Oberschenkelknochens (7), bestehend aus einem an die Markraumkonturen anpaßbaren, elastischen, spiralförmigen Schaft (5), einem am oberen Ende des Oberschenkelknochens (7) abstützbaren Kragen (2) und einem anschließenden einen Kugelkopf tragenden Halsstück (3), wobei der spiralförmige Schaft (5) aus mehreren Stäben besteht, dadurch gekennzeichnet, daß der Schaft als mehrgängige Schraubenfeder aus mehreren, jeweils eine Vielzahl von aufeinanderliegenden Windungen bildenden Stäben (14) oder als eingängige Schraubenfeder aus einem einzigen, eine Vielzahl von aufeinanderliegenden Windungen bildenden Stab (14) ausgebildet ist, wobei die Windungen gewickelt sind.

4. Hüftgelenksprothese nach einem der Ansprüche 1 bis 3, dadurch gekennzeichnet, daß die den Schaft (5) bildende Schraubenfeder einen Innendurchmesser aufweist, der zur Aufnahme eines Werkzeugs dient.

5. Hüftgelenksprothese nach einem der Ansprüche 1 bis 4, dadurch gekennzeichnet, daß das untere Schaftende (9) ein zur Schaftachse (S) paralleles Gewinde aufweist.

6. Hüftgelenksprothese nach Anspruch 5, dadurch gekennzeichnet, daß in das untere Schaftende (9) oder in eine an diesem angebrachte Gewindebuchse (17) ein Fixieranker (18) einschraubbar ist, der aus einer Komponente (21) zur Verankerung im Knochen (7) unterhalb des Schaftendes (9) und aus einem mit einem Gewinde versehenen Stab (Gewindestift 19) besteht, der in das Gewinde des Schaftes (5) bzw. in der Gewindebuchse (17) verstellbar einschraubbar und mit dem Werkzeug vom Kragen (2) her verstellbar ist.

7. Hüftgelenksprothese nach einem der Ansprüche 1 bis 6, dadurch gekennzeichnet, daß der Stab (14) bzw. die Stäbe (14) aus einer körperverträglichen Metallegierung besteht bzw. bestehen.

8. Hüftgelenksprothese nach Anspruch 1 oder 3, dadurch gekennzeichnet, daß der Querschnitt des Materials der Windungen bzw. des oder der Stäbe (14) bei der an den Kragen (2) anschließenden ersten Windung (25) oder den ersten Windungen (25) außen größer ist als innen, die Stäbe (14) also abwechselnd dünne und dicke, kontinuierlich ineinander übergehende Abschnitte aufweisen und die Länge der Abschnitte dem Umfang der jeweiligen Windung (25) entspricht.

9. Hüftgelenksprothese nach einem der Ansprüche 1 bis 8, dadurch gekennzeichnet, daß an der Unterseite des Kragens (2) und an der dem Hals (3) abgewandten Seite mehrere Rotationssicherungselemente (28) vorgesehen sind.

10. Hüftgelenksprothese nach Anspruch 9, dadurch gekennzeichnet, daß die Rotationssicherungselemente (28) als Stege ausgebildet sind.

## Claims

1. A hipjoint prosthesis (1) for cementfree implantation in the medullary cavity in the bone (7) of a femur and consisting of an elastic spiral shank (5) adaptable to the contours of the medullary cavity, a collar (2) which is able to bear against the top end of the bone (7) of the femur, and an adjoining neckpiece (3) carrying a ball head, where the spiral shank (5) consists of a number of rods lying spaced apart, characterized in that the shank is made as a multiple spiral spring of a number of rods (14) which form in each case a plurality of turns or as a single-start spiral spring of one single rod (14) which forms a plurality of turns, where over the whole length of the shank the clear space between the individual turns is less than the thickness of the rod (14) or rods (14), the turns being wound or machined from a solid shank.

2. A hipjoint prosthesis as in Claim 1, characterized in that the turns are machined by a metal-cutting operation or by electroerosion from the material of the shank.

3. A hipjoint prosthesis (1) for cementfree implantation in the medullary cavity in the bone (7) of a femur and consisting of an elastic spiral shank (5) adaptable to the contours of the medullary cavity, a collar (2) which is able to bear against the top end of the bone (7) of the femur, and an adjoining neckpiece (3) carrying a ball head, where the spiral shank (5) consists of a number of rods, characterized in that the shank is made as a multiple spiral spring of a number of rods (14) which form in each case a plurality of contiguous turns or as a single-start spiral spring of one single rod (14) which forms a plurality of contiguous turns, the turns being wound.

4. A hipjoint prosthesis as in one of the Claims 1 to 3, characterized in that the spiral spring forming the shank (5) exhibits an inner diameter which serves to receive a tool.

5. A hipjoint prosthesis as in one of the Claims 1 to 4, characterized in that the bottom end (9) of the shank exhibits one thread parallel with the axis (S) of the shank.

6. A hipjoint prosthesis as in Claim 5, characterized in that into the bottom end (9) of the shank or into a threaded bush (17) fitted to the latter a fixing anchor may be screwed, which consists of a component (21) for anchoring in the bone below the end (9) of the shank and of a rod (threaded pin 19) which is provided with a thread and may be screwed adjustably into the thread in the shank (5) or into the threaded bush (17) and may be adjusted by the tool from the collar (2).

7. A hipjoint prosthesis as in one of the Claims 1 to 6, characterized in that the rod (14) consists or the rods (14) consist of a bodily compatible metal alloy.

8. A hipjoint prosthesis as in Claim 1 or 3, characterized in that the cross-section of the material of the turns or respectively of the rod or rods (14) at the first turn (25) adjoining the collar (2) or the first turns (25) is greater on the outside than the inside, that is, the rods (14) exhibit alternately thin and thick portions which merge continuously into one another and the length of a portion corresponds with the circumference of the respective turn (25).

9. A hipjoint prosthesis as in one of the Claims 1 to 8, characterized in that on the underside of the collar (2) and on the side remote from the neck (3) a number of elements (28) are provided for securing against rotation.

10. A hipjoint prosthesis as in Claim 9, characterized in that the elements (28) for securing against rotation are made as webs.

## Revendications

1. Prothèse d'articulation de la hanche (1) pour l'implantation sans ciment dans le canal médullaire d'un fémur (7), prothèse consistant en un fût élastique (5) en forme de spirale, adaptable aux contours du canal médullaire, en une collerette (2) susceptible de prendre appui sur l'extrémité supérieure du fémur (7) et en une pièce de col (3) portant une tête sphérique et se raccordant à cette collerette, tandis que le fût (5) en forme de spirale est constitué de plusieurs barreaux espacés les uns des autres, prothèse caractérisée en ce que le fût est réalisé sous la forme d'un ressort à boudin à spires multiples à partir de plusieurs barreaux (14) formant chacun une pluralité de spires, ou bien sous la forme d'un ressort à boudin à spires simples à partir d'un barreau (14) unique formant une pluralité de spires, tandis que sur la longueur totale du fût, l'intervalle libre entre les différentes spires est plus réduit que l'épaisseur du barreau (14) ou des barreaux (14) et que les spires sont enroulées, ou bien façonnées dans la masse d'un fût plein.

2. Prothèse d'articulation de la hanche selon la revendication 1, caractérisée en ce que les spires sont façonnées dans la masse du matériau du fût par un processus d'usinage avec un enlèvement de copeaux ou par électroérosion.

3. Prothèse d'articulation de la hanche (1) pour l'implantation sans ciment dans le canal médullaire d'un fémur (7), prothèse consistant en un fût élastique (5) en forme de spirale, adaptable aux contours du canal médullaire, en une collerette (2) susceptible de prendre appui sur l'extrémité supérieure du fémur (7) et en une pièce de col (3) portant une tête sphérique et se raccordant à cette collerette, tandis que le fût en forme de spirale (5) est constitué par plusieurs barreaux, prothèse caractérisée en ce que le fût est réalisé sous la forme d'un ressort à boudin à spires multiples à partir de plusieurs barreaux (14) formant respectivement une pluralité de spires, ou bien sous la forme d'un ressort à boudin à spires simples à partir d'un unique barreau (14) formant une pluralité de spires, tandis que les spires sont enroulées.

4. Prothèse d'articulation de la hanche selon une des revendications 1 à 3, caractérisée en ce que le ressort à boudin formant le fût (5) a un diamètre interne qui sert à recevoir un outil.

5. Prothèse d'articulation de la hanche selon une des revendications 1 à 4, caractérisée en ce que l'extrémité inférieure (9) du fût comporte un filetage parallèle à l'axe (S) du fût.

6. Prothèse d'articulation de la hanche selon la revendication 5, caractérisée en ce que dans l'extrémité inférieure (9) du fût, ou dans une douille filetée (17) rapportée sur cette extrémité, un ancrage de fixation (18) peut être vissé, qui est constitué d'un composant (21) pour l'ancrage dans l'os (7) au-dessous de l'extrémité (9) du fût, et d'un barreau muni d'un filetage (broche fileté 19), qui est susceptible d'être vissé de façon réglable dans le filetage du fût (5) ou bien dans la douille filetée (17) et qui est réglable à l'aide de l'outil, à partir de la collerette.

7. Prothèse d'articulation de la hanche selon une des revendications 1 à 6, caractérisée en ce que le barreau (14) ou les barreaux (14) est ou sont en un alliage métallique compatible avec le corps humain.

8. Prothèse d'articulation de la hanche selon la revendication 1 ou la revendication 3, caractérisée en ce que la section transversale du matériau des spires ou bien du ou des barreaux (14), dans la première spire (25) ou dans les premières spires (25) se raccordant à la collerette (2) est plus grande à l'extérieur qu'à l'intérieur, tandis que les barreaux, ainsi alternativement minces et épais, comportent des tronçons passant en continu de l'un à l'autre, et que la longueur totale de ces deux tronçons correspond à la périphérie de la spire (25) considérée.

9. Prothèse d'articulation de la hanche selon une des revendications 1 à 8, caractérisée en ce que, sur le côté inférieur de la collerette, opposé au col (3) sont prévus plusieurs éléments (28) de blocage en rotation.

10. Prothèse d'articulation de la hanche selon la revendication 9, caractérisée en ce que les éléments (28) de blocage en rotation revêtent la forme de nervures.
